# EUROPÄISCHE PATENTSCHRIFT

(11) **EP 1 355 636 B1**
(45) Veröffentlichungstag und Bekanntmachung des Hinweises auf die Patenterteilung: **14.05.2008**
(21) Anmeldenummer: 02715442.6
(22) Anmeldetag: 17.01.2002
(51) Int. Cl.: A61K 9/70

(54) **TRANSDERMALES THERAPEUTISCHES SYSTEM FÜR DIE VERABREICHUNG CARBOXYLGRUPPENHALTIGER, NICHTSTEROIDALER ANTIPHLOGISTIKA, SOWIE VERFAHREN ZU SEINER HERSTELLUNG**
TRANSDERMAL THERAPEUTIC SYSTEM FOR ADMINISTERING NON-STEROIDAL ANTIPHLOGISTIC AGENTS CONTAINING CARBOXYL GROUPS, AND A METHOD FOR THE PRODUCTION OF THE SAME
SYSTEME THERAPEUTIQUE TRANSDERMIQUE POUR L'ADMINISTRATION D'ANTIPHLOGISTIQUES NON STEROIDIQUES, CONTENANT UN GROUPE CARBOXYLIQUE, ET SON PROCEDE DE PRODUCTION

(30) Priorität: 30.01.2001 DE 10103860
(43) Veröffentlichungstag der Anmeldung: 29.10.2003
(73) Patentinhaber: LTS LOHMANN Therapie-Systeme AG, 56626 Andernach (DE)
(72) Erfinder: BRACHT, Stefan, 56299 Ochtendung (DE)
(74) Vertreter: Flaccus, Rolf-Dieter
(86) Internationale Anmeldenummer: PCT/EP2002/000417
(87) Internationale Veröffentlichungsnummer: WO 2002/060418

(56) Entgegenhaltungen:
- EP-A- 0 446 636
- EP-A- 0 965 626
- DE-A- 19 830 649
- DE-A- 19 918 106
- US-A- 5 125 995
- PATENT ABSTRACTS OF JAPAN vol. 016, no. 283 (C-0955), 24. Juni 1992 (1992-06-24) & JP 04 074119 A (NITTO DENKO CORP), 9. März 1992 (1992-03-09) in der Anmeldung erwähnt

## Beschreibung

Die vorliegende Erfindung betrifft transdermale therapeutische Systeme (TTS), welche für die Verabreichung carboxylgruppenhaltiger, nichtsteroidaler Antiphlogistika bestimmt sind. Die Erfindung betrifft insbesondere TTS der genannten Art, welche eine Matrix auf der Basis von carboxylgruppenhaltigen Polyacrylathaftklebern aufweisen.
Des weiteren umfaßt die vorliegende Erfindung auch Verfahren zur Herstellung von TTS der genannten Art.

Wirkstoffe aus der Gruppe der carboxylgruppenhaltigen nichtsteroidalen Antirheumatika bzw. Antiphlogistika (dt.: "NSAR", engl.: "NSAID") finden breite Anwendung bei der Behandlung von Entzündungen, entzündlichen Gelenkerkrankungen, rheumatischen Krankheitsbildern und der dadurch bedingten Schmerzzustände.
Zu dieser Wirkstoffgruppe zählen einerseits Vertreter der Essigsäurederivate, wie z. B. Indomethacin, Acemetacin, Tolmetin, Diclofenac und Lonazolac, und andererseits Vertreter der Propionsäurederivate (Profene), wie z. B. Ibuprofen, Flurbiprofen, Fenoprofen, Ketoprofen, Naproxen und Tiaprofen.
Da durch die orale Aufnahme dieser Wirksubstanzen häufig Nebenwirkungen, wie z. B. Magenbeschwerden und Blutungen im Gastrointestinalbereich, verursacht werden, ist die topische oder transdermale Verabreichung grundsätzlich zu bevorzugen. Dies kann mit Hilfe von Gelen, Cremes, Salben oder auf der Haut haftklebenden Pflastern erfolgen. Letztere haben den zusätzlichen Vorteil, daß sie - anders als z. B. Salben - längere Zeit am Applikationsort verbleiben können und einfacher anzuwenden sind.
TTS mit einem Gehalt an NSAR sind beispielsweise aus DE 198 30 649 A1 bekannt. Auf diese Veröffentlichung, wie auch auf die dort gemachten Ausführungen zum Stand der Technik, wird hier ausdrücklich Bezug genommen.
Aus DE 198 30 649 A1 geht hervor, daß mit einer Kombination von NSAID, insbesondere Ketoprofen, Flurbiprofen oder Ibuprofen, mit Fettsäuren als Permeationsbeschleunigern und einer carboxylgruppenhaltigen Klebermatrix hohe Freisetzungsraten erzielt werden können.

Allerdings wurde nun durch weitere Untersuchungen festgestellt, daß die Matrixformulierungen gemäß den Beispielen 1, 2 und insbesondere 3 aus DE 198 30 649 A1 durch den hohen Gehalt an Wirkstoff und weichmachenden Fettsäuren eine sehr weiche Konsistenz und höchst unbefriedigende kohäsive Eigenschaften aufweisen. Zudem wurden im frisch hergestellten Zustand Probleme bezüglich der Verankerung der wirkstoffhaltigen Matrixschicht speziell auf dem als Rückschicht verwendeten Polyethylenterephthalat (PET) beobachtet, und zwar sowohl bei der Verwendung von PET-Folien als auch von elastischen Geweben aus PET. Dies ist gerade deshalb von Nachteil, da PET wegen seiner Barriere-Eigenschaft gegenüber Wirkstoffen sowie aufgrund seiner mechanischen Strapazierfähigkeit das für Rückschichten am meisten bevorzugte Material ist. Folglich bedeutet eine schlechte Verankerung der Wirkstoffmatrix auf derartigen PET-Materialien entsprechend große Einschränkungen bei der Nutzung der in DE 198 30 649 A1 offenbarten Formulierungen zur Herstellung von TTS, welche carboxylgruppenhaltige NSAR enthalten.

Um die Kohäsion der Haftkleberschicht und zugleich die Freisetzung des Wirkstoffs Ibuprofen zu verbessern, wurde in JP 4074119 A der Zusatz eines Alkalihydroxids vorgeschlagen. Der Zusatz einer permeationsfördernden Fettsäure ist dabei allerdings nicht vorgesehen.

Aufgabe der vorliegenden Erfindung war es deshalb, transdermale therapeutische Systeme bereitzustellen, welche für die Verabreichung der genannten Wirkstoffe geeignet sind, und welche sich durch die in DE 198 30 649 A1 beschriebenen vorteilhaften Eigenschaften auszeichnen, ohne jedoch die vorstehend beschriebenen Nachteile aufzuweisen.
Darüber hinaus wird gefordert, daß die Herstellung der NSAR-enthaltenden Polymermatrix im nicht-wässrigen Milieu, d.h. in organischen Lösungsmitteln möglich sein muß. Dies ist deshalb von Bedeutung, da die für die Ausführung der Erfindung in Betracht kommenden carboxylgruppenhaltigen Haftkleber in überwiegender Mehrzahl nur in organischer Lösung verarbeitet werden können.

Die Lösung besteht darin, daß bei einem TTS mit den Merkmalen des Oberbegriffs des Anspruchs 1 die wirkstoffhaltige Matrix auf der Basis eines carboxylgruppenhaltigen Polyacrylathaftklebers hergestellt ist, und daß sie einen Gehalt an Ölsäure und an Kaliumionen aufweist, wobei der Mengenanteil der Kaliumionen im Bereich von 5 bis 50 % liegt, bezogen auf das Äquivalentgewicht der Summe aller carboxylgruppenhaltigen Matrixbestandteile.

Es wurde nämlich überraschenderweise gefunden, daß allein durch die Verwendung von Ölsäure als permeationsfördernder Fettsäure und von Kaliumhydroxid als Alkalihydroxid die Herstellung der Wirkstoffmatrix in nicht-wässrigem Milieu, tpyischerweise in Ethylacetat, ermöglicht wird. Eine Ausfällung des Fettsäuresalzes als unlöslicher Niederschlag unterbleibt in diesem Fall. Das Ausbleiben einer derartigen Niederschlagsbildung ist vermutlich auf die spezifischen Eigenschaften des Ölsäure-Kaliumsalzes zurückzuführen, welches durch den erfindungsgmäßen Zusatz von Ölsäure und Kaliumionen gebildet wird. Bei der Verwendung anderer Alkali-Fettsäuresalze kommt es in Gegenwart eines organischen Lösungsmittel zu Niederschlagsbildung (siehe Beispiele 1 bis 8 und Tab. 1).

Aufgrund dieser vorteilhaften Eigenschaft, die sich aus der Kombination von Ölsäure mit Kaliumionen ergibt, können für die Herstellung der erfindungsgemäßen Matrixschichten grundsätzlich alle carboxylgruppenhaltigen Haftkleber verwendet werden, die in organischer Lösung verarbeitet werden, z. B. die unter der Bezeichnung "Durotak" von der Fa. National Starch vertriebenen Haftkleber.
Da bei den erfindungsgemäßen TTS eine Ausfällung von Ölsäure in der Matrixschicht zuverlässig verhindert werden kann, zeichnen sich diese TTS durch eine sehr homogene Matrixschicht aus; vorzugsweise besteht diese Matrixschicht nur aus einer einzigen Phase, d. h. sie stellt keine Emulsion oder Suspension oder mehrphasige Polymermischung dar.

Die erfindungsgemäßen Matrixschichten weisen ferner gegenüber DE 198 30 649 A1 eine deutlich verbesserte Kohäsion der haftklebenden Matrixschicht und eine einwandfreie Verankerung auf Rückschichten aus PET-Materialien auf. Gegenüber JP 4074119 ergibt sich eine wesentliche Verbesserung der Hautpermeation des Wirkstoffs aufgrund der permeationsfördernden Wirkung der enthaltenen Ölsäure.

Als carboxylgruppenhaltige Polyacrylathaftkleber zur Herstellung der erfindungsgemäßen Wirkstoffmatrix werden bevorzugt carboxylgruppenhaltige Haftkleber verwendet, die unter der Bezeichnung "Durotak" von der Fa. National Starch vertrieben werden, insbesondere der Haftkleber Durotak 387-2353. Daneben eignen sich grundsätzlich auch andere Polyacrylatkleber, die durch radikalische Polymerisation von Acryl- und/oder Methacrylsäure und ihren Derivaten (z. B. 2-Ethylhexylacrylat) hergestellt sind. Zusätzlich können solche Polymere auch weitere Comonomere enthalten, z. B. Vinylacetat. Im Einzelfall können auch geeignete Mischungen verschiedener carboxylgruppenhaltiger Polyacrylathaftkleber zum Einsatz kommen.
Das Flächengewicht der Matrixschicht liegt vorzugsweise im Bereich von 30-120 g/m², wobei ein Flächengewicht im Bereich von 50-80 g/m² besonders bevorzugt wird.

Die in den erfindungsgemäßen Matrixschichten enthaltenen Kaliumionen werden vorzugsweise als Kaliumhydroxid zugesetzt. Da die erfindungsgemäßen Formulierungen mindestens drei carboxylgruppenhaltige Bestandteile aufweisen, nämlich den Wirkstoff, Ölsäure, sowie den carboxylgruppenhaltigen Haftkleber, läßt sich nur sehr schwer ermitteln, mit welcher dieser carboxylgruppenhaltigen Komponenten es zu einer Salzbildung kommt, und in welchem Verhältnis. Deshalb wurde auf eine experimentelle Untersuchung dieser Salzbildung verzichtet.

Statt dessen wird vorgezogen, die Menge des enthaltenen Kaliumhydroxids in Prozentanteilen des Äquivalentgewichts der Summe der carboxylgruppenhaltigen Bestandteile der Matrix anzugeben (siehe Beispiel 9). Eine Menge von 100 % Kaliumhydroxydäquivalenten entspricht dann der Neutralisation sämtlicher carboxylgruppenhaltiger Matrixbestandteile.
Es wurde die Beobachtung gemacht, daß eine deutliche Erhöhung der Kohäsion des Matrixfilms erst eintritt, wenn mindestens eine Menge von 5 %, besser 10 % Kaliumhydroxidäquivalenten zugesetzt wird. Liegt andererseits die zugesetzte KOH-Menge über einem Wert 50 % Kaliumhydroxidäquivalenten, so verliert die Matrixschicht bzw. der Matrixfilm stark an Klebrigkeit. Gleichzeitig nimmt die Hydrophilie der Matrix stark zu, so daß der Matrixfilm beim Tragen auf der Haut durch Aufnahme von Hautfeuchtigkeit weiß anläuft und an Klebkraft verliert.

Bevorzugt wird KOH in einer Menge von 10 bis 40 % Kaliumhydroxidäquivalenten zugesetzt, wobei optimale Resultate bei ca. 30 % Kaliumhydroxidäquivalenten erzielt wurden.

Der Gehalt an Ölsäure kann zwischen 5 und 25 Gew.-% betragen, bezogen auf die Matrixschicht; vorzugsweise wird Ölsäure in einem Anteil von 10-20 Gew.-% zugesetzt.

Als Wirkstoffe aus der Gruppe der carboxylgruppenhaltigen NSAR werden bevorzugt Ibuprofen, Flurbiprofen, Fenoprofen, Ketoprofen, Naproxen und Tiaprofen verwendet, wobei Ibuprofen besonders bevorzugt ist; es können aber auch andere Vertreter dieser Wirkstoffgruppe eingesetzt werden, beispielsweise die eingangs genannten Wirkstoffe. Bevorzugt liegt der Wirkstoff im wesentlichen als (S)-Enantiomer vor. Die Wirkstoffkonzentration in der Matrix beträgt vorzugsweise 5 bis 25 Gew.-%, besonders bevorzugt 10 bis 20 Gew.-%.

Gemäß einer besonderen Ausführungsform der Erfindung ist vorgesehen, daß der Haftkleber durch Aluminiumionen quervernetzt vorliegt. Als Vernetzungsreagens wird vorzugsweise Aluminiumacetylacetonat verwendet. Durch die Vernetzung wird die Kohäsion der Kleberschicht zusätzlich verbessert. Aluminiumacetylacetonats wird vorzugsweise in einer Konzentration von 0,01 bis 0,2 Gew.-% zugesetzt, wobei der Konzentrationsbereich von 0.05 bis 0,1 Gew.-% besonders bevorzugt ist. Die genannten Prozentangaben sind als Aluminium-Ionen-Konzentration in der wirkstoffhaltigen Klebermatrix berechnet.

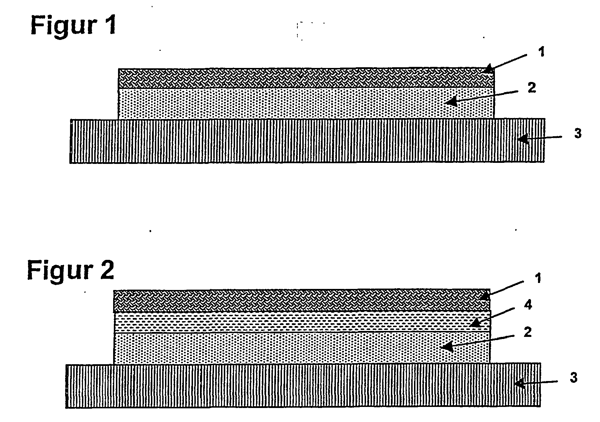

Die vorliegende Erfindung umfaßt ferner auch solche Ausführungsformen, bei welchen zwischen der wirkstoffhaltigen Matrixschicht des TTS und dessen Rückschicht eine wasserdampfsperrende Zwischenschicht angebracht ist, die beim Tragen auf der Haut okklusiv wirkt (siehe Fig. 2). Diese Zwischenschicht ist vorzugsweise aus Kohlenwasserstoffpolymeren aufgebaut, z. B. aus Polyisobutylen, Polybutadien, Polyisopren oder Blockcopolymeren des Styrols mit Butadien oder Isopren. Das Flächengewicht der Zwischenschicht liegt im Bereich von 10 bis 100 g/m², vorzugsweise im Bereich von 20 bis 80 m².
Das Anbringen einer solchen Zwischenschicht ist insbesondere dann von Vorteil, wenn die Rückschicht aus wasserdampfdurchlässigem Material besteht.

Als Material für die Rückschicht der erfindungsgemäßen TTS wird bevorzugt Polyethylenterephthalat(PET)-Folie, besonders bevorzugt aber längs- und querelastisches, luft- und wasserdampfdurchlässiges PET-Gewebe verwendet. Aber auch andere, dem Fachmann bekannte Polyester-Folien oder -Gewebe können verwendet werden, wobei Materialien mit elastischen Eigenschaften bevorzugt werden.

Für die ablösbare Schutzschicht kommen dehäsiv ausgerüstete (z. B. durch Silikonisierung) Kunststoffolien in Betracht, beispielsweise aus Polyethylen, Polyethylenterephthalat (PET) oder ähnlichen Materialien.

Der Aufbau der erfindungsgemäßen TTS wird anhand der Fig. 1 und 2 beispielhaft erläutert. Es handelt sich dabei um schematische, vereinfachte Zeichnungen, welche jeweils ein TTS in Schnittdarstellung zeigen.

Fig. 1 zeigt ein TTS, bestehend aus einer Rückschicht (1) und einer homogenen, wirkstoffhaltigen Matrixschicht (2). Die hautzugewandte Seite der Matrixschicht (2) ist mit einer wiederablösbaren Schutzschicht (3) bedeckt.

Dieser bevorzugte Aufbau entspricht demjenigen, der in Beispiel 9 beschrieben wird.

Fig. 2 zeigt einen weiteren, bevorzugten Aufbau eines erfindungsgemäßen TTS, der ebenfalls eine Rückschicht (1), eine homogene, wirkstoffhaltige Matrixschicht (2) und eine wiederablösbare Schutzschicht (3) aufweist. Zusätzlich befindet sich zwischen Matrixschicht (2) und Rückschicht (1) eine wasserdampfsperrende Zwischenschicht (4).

### Beispiele 1 bis 8:

### Löslichkeit von Alkali-Fettsäuresalzen

Die Versuche wurden wie folgt durchgeführt: Die in Tabelle 1 bezeichnete Fettsäure wurde in Ethylacetat in einer Konzentration von 10 Gew.-% gelöst. Anschließend wurde diese Lösung mit 2 Tropfen einer Lösung des angegebenen Alkalihydroxids in Methanol (10 Gew.-%) versetzt. Es wurde festgehalten, ob an der Eintropfstelle ein weißer Niederschlag (N) auftrat, oder ob die Lösung klar (K) blieb. Die Ergebnisse sind in Tab. 1 dargestellt.

**Tabelle 1:**

| | Laurinsäure | | Myristinsäure | | Stearinsäure | | Ölsäure | |
|---|---|---|---|---|---|---|---|---|
| NaOH | Bsp. 1 | N | Bsp. 2 | N | Bsp. 3 | N | Bsp. 4 | N |
| KOH | Bsp. 5 | N | Bsp. 6 | N | Bsp. 7 | N | Bsp. 8 | K |

Unter den bei der Herstellung von TTS gebräuchlichsten Fettsäuren erwies sich allein das Kaliumsalz der Ölsäure als löslich in Ethylacetat (Beispiel 8).

### Beispiel 9:

### Herstellung eines TTS mit dem Wirkstoff Ibuprofen

### Zusammensetzung:

| | Gew.-% | Äquivalentgewicht an KOH |
|---|---|---|
| | | (in mg KOH/g Substanz) |
| Ibuprofen | 16,63 | 270 |
| Ölsäure | 16,63 | 199 |
| Kaliumhydroxid | 3,16** | - |
| Aluminum* | 0,1 | - |
| Durotak 387-2353 | 63,48 | 49 |
| Summe: | 100,00 | |

| | | |
|---|---|---|
| Die Prozentangaben beziehen sich auf das Matrixgewicht. *vernetzend an das haftklebende Polymer gebunden. **Dieser Anteil entspricht ca. 29 % des Äquivalenzgewichts der Summe aller carboxylgruppenhaltiger Bestandteile. | | |

### Herstellung:

Zur Herstellung des Beispiels 9 wird zunächst die Ölsäure in die Durotak-Haftkleberlösung (Durotak 387-2353; Fa. National Starch) eingerührt. Danach wird Kaliumhydroxid in Form einer 10 %igen (Gew.-%) Lösung in Methanol unter Rühren langsam zugefügt. Schließlich wird Ibuprofen in diesem Ansatz aufgelöst und das pulverförmige Vernetzungsreagenz Aluminiumacetylacetonat hinzugefügt und bis zur Auflösung eingerührt. Zur Einstellung einer für die Beschichtung geeigneten Viskosität wird zu der fertigen Lösung nach Bedarf Ethylacetat zugefügt.

Diese Kleberlösung wird auf eine silikonisierte Polyesterfolie (PET, 100 µm) beschichtet. Die Beschichtungsdicke wird so eingestellt, daß nach 10minütiger Trocknung bei 60 °C in einem Abluftofen ein Film mit einem Flächengewicht von 60 bis 80 g/m² erhalten wird. Der getrocknete Kleberfilm wird mit einem längs- und querelastischen PET-Gewebe (Fa. KOB Karl Otto Braun) als Rückschicht ausgestattet. Anschließend werden aus diesem Laminat einzelne Pflaster geeigneter Form und Größe ausgestanzt.

## Patentansprüche

1. Transdermales therapeutisches System (TTS) mit einem Gehalt an mindestens einem Wirkstoff aus der Gruppe der carboxylgruppenhaltigen nichtsteroidalen Antirheumatika, welches eine Rückschicht (1), eine wirkstoffhaltige Matrixschicht (2) auf der Basis eines carboxylgruppenhaltigen Polyacrylathaftklebers sowie eine wiederablösbare Schutzschicht (3) aufweist, **dadurch gekennzeichnet daß** die Matrixschicht (2) einen Gehalt an Ölsäure aufweist und einen Gehalt an Kaliumionen, wobei der Mengenanteil der Kaliumionen im Bereich von 5 - 50 % liegt, bezogen auf das Äquivalenzgewicht der Summer aller carboxylgruppenhaltigen Matrixbestandteile.

2. TTS nach Anspruch 1, **dadurch gekennzeichnet, daß** der Mengenanteil der Kaliumionen im Bereich von 10 bis 40 % liegt, bezogen auf das Äquivalentgewicht der Summe aller carboxylgruppenhaltigen Matrixbestandteile.

3. TTS nach Anspruch 1 oder 2, **dadurch gekennzeichnet, daß** der Gehalt an Ölsäure in der Matrixschicht 5 bis 25 Gew.-% beträgt, vorzugsweise 10-20 Gew.-%.

4. TTS nach einem der Ansprüche 1 bis 3, **dadurch gekennzeichnet, daß** es mindestens einen Wirkstoff aus der Gruppe der Propionsäurederivate enthält, wobei der/die Wirkstoff(e) bevorzugt aus der Ibuprofen, Flurbiprofen, Fenoprofen, Ketoprofen, Naproxen und Tiaprofen umfassenden Gruppe ausgewählt ist/sind, und wobei Ibuprofen besonders bevorzugt ist.

5. TTS nach einem der Ansprüche 1 bis 4, **dadurch gekennzeichnet, daß** es mindestens einen Wirkstoff aus der Gruppe der Essigsäurederivate enthält, wobei der/die Wirkstoff(e) bevorzugt aus der Indomethacin, Acemetacin, Tolmetin, Diclofenac und Lonazolac umfassenden Gruppe ausgewählt ist/sind.

6. TTS nach Anspruch 4 oder 5, **dadurch gekennzeichnet, daß** der Wirkstoff im wesentlichen als (S)-Enantiomer vorliegt.

7. TTS nach einem der Ansprüche 1 bis 6, **dadurch gekennzeichnet, daß** die Wirkstoffkonzentration in der wirkstoffhaltigen Matrix 5 bis 25 Gew.-% beträgt, vorzugsweise 10 bis 20 Gew.-%.

8. TTS nach einem der Ansprüche 1 bis 7, **dadurch gekennzeichnet, daß** die carboxylgruppenhaltige haftklebende Polymermatrix durch Aluminiumionen quervernetzt vorliegt.

9. TTS nach einem oder mehreren der vorangehenden Ansprüche, **dadurch gekennzeichnet, daß** die Matrixschicht homogen aufgebaut ist und nur aus einer einzigen Phase besteht.

10. TTS nach einem oder mehreren der vorangehenden Ansprüche, **dadurch gekennzeichnet, daß** die Rückschicht aus Polyesterfolie besteht, vorzugsweise aus Polyethylenterephthalat-Folie, besonders bevorzugt aus elastischem Polyestergewebe.

11. TTS nach einem oder mehreren der vorangehenden Ansprüche, **dadurch gekennzeichnet, daß** zwischen der wirkstoffhaltigen Matrixschicht und der Rückschicht eine wasserdampfsperrende Zwischenschicht angebracht ist, welche vorzugsweise aus Kohlenwasserstoffpolymeren aufgebaut ist, wobei das Flächengewicht der Zwischenschicht 10 bis 100 g/m², besonders bevorzugt 20 bis 80 m² beträgt.

12. Verfahren zur Herstellung eines transdermalen therapeutischen Systems (TTS) mit einem Gehalt an mindestens einem Wirkstoff aus der Gruppe der carboxylgruppenhaltigen nichtsteroidalen Antirheumatika, welches eine Rückschicht, eine wirkstoffhaltige Matrixschicht auf der Basis eines carboxylgruppenhaltigen Polyacrylathaftklebers und eine wiederablösbare Schutzschicht aufweist, **gekennzeichnet durch** folgende Schritte:
a) Bereitstellen des carboxylgruppenhaltige Polyacrylathaftklebers in organischer Lösung;
b) Zugabe von 5 bis 25 Gew.-% Ölsäure, unter Rühren;
c) Zugabe von KOH in 10%iger (Gew.-%) methanolischer Lösung unter Rühren, wobei die Menge an KOH im Bereich von 5 bis 50 % liegt, bezogen auf das Äquivalentgewicht der Summe aller carboxylgruppenhaltigen Matrixbestandteile;
d) Zugabe des Wirkstoffs aus der Gruppe der carboxylgruppenhaltigen nichtsteroidalen Antirheumatika, der unter Rühren in dem Haftkleber-Ansatz gelöst wird;
e) Zugabe von Aluminiumacetylacetonat in Pulverform und Rühren bis zur vollständigen Auflösung, wobei die Konzentration des Aluminiumacetylacetonats 0,01 bis 0,2 Gew.-%, vorzugsweise 0.05 bis 0,1 Gew.-% beträgt, jeweils berechnet als Aluminium-Ionen in der wirkstoffhaltigen Klebermatrix;
f) Beschichten dieser Haftkleberlösung auf eine silikonisierte Polyesterfolie und Trocknen zur Entfernung der Lösemittel;
g) Bedecken der getrockneten Schicht mit einer elastischen Polyesterfolie oder einem elastischen Polyestergewebe als Rückschicht;
h) Ausstanzen einzelner TTS.

13. Verfahren nach Anspruch 12, **dadurch gekennzeichnet, daß** nach Schritt f) die getrocknete Haftkleberschicht mit einer Zwischenschicht aus Kohlenwasserstoffpolymeren bedeckt wird, auf welche nachfolgend die Rückschicht aufgebracht wird (Schritt g), wobei die Kohlenwasserstoffpolymeren vorzugsweise aus der Gruppe von Polyisobutylen, Polybutadien, Polyisopren oder Blockcopolymeren des Styrols mit Butadien oder Isopren ausgewählt sind.

## Claims

1. Transdermal therapeutic system (TTS) with a content of at least one active substance from the group of the carboxyl group-containing non-steroidal antirheumatics, which has a backing layer (1), an active substance-containing matrix layer (2) based on a carboxyl group-containing polyacrylate pressure-sensitive adhesive, and a detachable protective layer (3), **characterised in that** said matrix layer (2) has a content of oleic acid and a content of potassium ions, the constituent amount of the potassium ions being in the range of from 5 to 50%, relative to the equivalent weight of the sum of all carboxyl group-containing matrix components.

2. TTS according to claim 1, **characterized in that** the constituent amount of the potassium ions is in the range of from 10 to 40%, relative to the equivalent weight of the sum of all carboxyl group-containing matrix components.

3. TTS according to claim 1 or 2, **characterized in that** the content of oleic acid in the matrix layer is 5 to 25%-wt., preferably 10-20%-wt.

4. TTS according to claim 1 to 3, **characterized in that** it contains at least one active substance from the group of propionic acid derivatives, with the active substance(s) preferably being selected from the group comprising ibuprofen, flurbiprofen, fenoprofen, ketoprofen, naproxen, and tiaprofen, and with ibuprofen being particularly preferred.

5. TTS according to any one of claims 1 to 4, **characterized in that** it contains at least one active substance of the group of acetic acid derivatives, with the active substance(s) preferably being selected from the group comprising indomethacin, acemetacin, tolmetin, diclofenac and lonazolac.

6. TTS according to claim 4 or 5, **characterized in that** the active substance is present substantially as (S)-enantiomer.

7. TTS according to any one of claims 1 to 6, **characterized in that** the active substance concentration in the active substance-containing matrix amounts to 5 to 25%-wt., preferably 10 to 20%-wt.

8. TTS according to any one of claims 1 to 7, **characterized in that** the carboxyl group-containing pressure-sensitive adhesive polymer matrix is present cross-linked by aluminium ions.

9. TTS according to one or more of the preceding claims, **characterized in that** the matrix layer is of a homogenous structure and consists of a single phase only.

10. TTS according to one or more of the preceding claims, **characterized in that** the backing layer consists of polyester film, preferably of polyethylene terephthalate film, with particular preference of elastic polyester fabric.

11. TTS according to one or more of the preceding claims, **characterized in that** between the active substance-containing matrix layer and the backing layer there is arranged a water vapour-blocking intermediate layer which is preferably made up of hydrocarbon polymers, with the weight per unit area of the intermediate layer being 10 to 100 g/m², with particular preference 20 to 80 m².

12. Process for the production of a transdermal therapeutic system (TTS) having a content of at least one active substance from the group of the carboxyl group-containing non-steroidal antirheumatics, which system has a backing layer, an active substance-containing matrix layer based on a carboxyl group-containing polyacrylate pressure sensitive adhesive, and a detachable protective layer, **characterized by** the following steps:
a) providing the carboxyl group-containing polyacrylate pressure-sensitive adhesive in organic solution;
b) adding 5 to 25%-wt. of oleic acid, by stirring;
c) adding KOH in 10% solution (%-wt.) of methanolic solution under stirring, with the amount of KOH being in the range of from 5 to 50%, relative to the equivalent weight of the sum of all carboxyl group-containing matrix components;
d) adding the active substance from the group of the carboxyl group-containing non-steroidal antirheumatics, which active substance is dissolved in the pressure-sensitive adhesive preparation by stirring;
e) adding aluminium acetylacetonate in pulverulent form and stirring until complete solution occurs, with the concentration of the aluminium acetylacetonate being 0.01 to 0.2%-wt, preferably 0.05 to 0.1%-wt, each calculated as aluminium ions in the active substance-containing adhesive matrix;
f) coating the pressure-sensitive adhesive solution to a siliconized polyester film, and drying in order to remove the solvents;
g) covering the dried layer with an elastic polyester film or an elastic polyester fabric as backing layer;
h) punching out individual TTS.

13. Process according to claim 12, **characterized in that** following step f), the dried layer of pressure-sensitive adhesive is covered with an intermediate layer of hydrocarbon polymers to which subsequently the backing layer is applied (step g), with the hydrocarbon polymers preferably being selected from the group of polyisobutylene, polybutadiene, polyisoprene or block copolymers of styrene with butadiene or isoprene.

## Revendications

1. Système thérapeutique transdermique (TTS) ayant une teneur en au moins une substance active issue du groupe formé par les agents antirhumatismaux non stéroïdiens contenant des groupes carboxyle, lequel présente une couche de support (1), une couche de matrice (2) contenant une substance active à base d'une colle auto-adhésive polyacrylate contenant des groupes carboxyle ainsi qu'une couche protectrice (3) amovible, **caractérisé en ce que** la couche de matrice (2) présente une teneur en acide oléique et une teneur en ions potassium, la proportion quantitative des ions potassium se trouvant dans la plage de 5-50%, par rapport au poids équivalent de la somme de tous les composants de matrice contenant des groupes carboxyle.

2. TTS selon la revendication 1, **caractérisé en ce que** la proportion quantitative des ions potassium se trouve dans la plage de 10 à 40%, par rapport au poids équivalent de la somme de tous les composants de matrice contenant des groupes carboxyle.

3. TTS selon la revendication 1 ou 2, **caractérisé en ce que** la teneur en acide oléique dans la couche de matrice est de 5 à 25% en poids, de préférence de 10 à 20% en poids.

4. TTS selon l'une quelconque des revendications 1 à 3, **caractérisé en ce qu'**il contient au moins une substance active issue du groupe des dérivés de l'acide propionique, la ou les substances actives étant choisies de préférence dans le groupe comprenant l'ibuprofène, le flurbiprofène, le fénoprofène, le kétoprofène, le naproxène, et le tiaprofène, et l'ibuprofène étant particulièrement préféré.

5. TTS selon l'une quelconque des revendications 1 à 4, **caractérisé en ce qu'**il contient au moins une substance active issue du groupe des dérivés de l'acide acétique, la ou les substances actives étant choisies de préférence dans le groupe comprenant l'indométhacine, l'acémétacine, la tolmétine, le diclofénac, et le lonazolac.

6. TTS selon la revendication 4 ou 5, **caractérisé en ce que** la substance active existe essentiellement sous forme de (S)-énantiomère.

7. TTS selon l'une quelconque des revendications 1 à 6, **caractérisé en ce que** la concentration de la substance active dans la matrice contenant la substance active est de 5 à 25% en poids, de préférence de 10 à 20% en poids.

8. TTS selon l'une quelconque des revendications 1 à 7, **caractérisé en ce que** la matrice polymère auto-adhésive contenant des groupes carboxyle existe à l'état réticulé par des ions aluminium.

9. TTS selon l'une ou plusieurs des revendications précédentes, **caractérisé en ce que** la couche de matrice est construite de manière homogène et n'est constituée que d'une seule phase.

10. TTS selon l'une ou plusieurs des revendications précédentes, **caractérisé en ce que** la couche de support est constituée d'une feuille de polyester, de préférence une feuille de poly(téréphtalate d'éthylène), de manière particulièrement préférée un tissu élastique à base de polyester.

11. TTS selon l'une ou plusieurs des revendications précédentes, **caractérisé en ce qu'**entre la couche de matrice contenant la substance active et la couche de support, une couche intermédiaire imperméable à la vapeur d'eau est appliquée, laquelle est construite de préférence à partir de polymères hydrocarbonés, le grammage de la couche intermédiaire étant de 10 à 100 g/m², de manière particulièrement préférée de 20 à 80 g/m².

12. Procédé de préparation d'un système thérapeutique transdermique (TTS) ayant une teneur en au moins une substance active issue du groupe formé par les agents antirhumatismaux non stéroïdiens contenant des groupes carboxyle, lequel présente une couche de support, une couche de matrice contenant la substance active à base d'une colle auto-adhésive polyacrylate contenant des groupes carboxyle, et une couche protectrice amovible, **caractérisé par** les étapes suivantes:
a) préparation de la colle auto-adhésive polyacrylate contenant des groupes carboxyle dans une solution organique;
b) ajout de 5 à 25% en poids d'acide oléique, sous agitation;
c) ajout de KOH dans une solution méthanolique à 10% (% en poids) sous agitation, la quantité de KOH se trouvant dans la plage de 5 à 50%, par rapport au poids équivalent de la somme de tous les composants de matrice comprenant des groupes carboxyle;
d) ajout de la substance active issue du groupe des agents antirhumatismaux non stéroïdiens contenant des groupes carboxyle, qui est dissoute sous agitation dans le mélange de colle auto-adhésive;
e) ajout d'acétylacétonate d'aluminium sous forme de poudre et agitation jusqu'à dissolution complète, la concentration de l'acétylacétonate d'aluminium étant de 0,01 à 0,2% en poids, de préférence de 0,05 à 0,1% en poids, à chaque fois calculé en ions aluminium dans la matrice de colle contenant la substance active;
f) enduction de cette solution de colle auto-adhésive sur une feuille de polyester siliconée et séchage pour éliminer le solvant;
g) recouvrement de la couche séchée par une feuille de polyester élastique ou un tissu de polyester élastique en tant que couche de support;
h) matriçage de TTS individuels.

13. Procédé selon la revendication 12, **caractérisé en ce que**, après l'étape f), la couche de colle auto-adhésive séchée est recouverte d'une couche intermédiaire de polymères hydrocarbonés, sur laquelle la couche de support est appliquée par la suite (étape g), les polymères hydrocarbonés étant choisis de préférence dans le groupe formé par le polyisobutylène, le polybutadiène, le polyisoprène ou les copolymères séquencés du styrène avec le butadiène ou l'isoprène.
